(19) Europäisches Patentamt
European Patent Office
Office européen des brevets

(11) Publication number: **0 418 013 A2**

(12) **EUROPEAN PATENT APPLICATION**

(21) Application number: 90309872.1

(51) Int. Cl.5: C07F 3/02, C07B 41/00

(22) Date of filing: 10.09.90

(30) Priority: 11.09.89 US 405561

(43) Date of publication of application: 20.03.91 Bulletin 91/12

(84) Designated Contracting States: AT BE CH DE DK ES FR GB IT LI LU NL SE

(71) Applicant: ARCH DEVELOPMENT CORPORATION
1101 East 58th Street
Chicago, Illinois 60637(US)

(72) Inventor: Eaton, Philip E.
5532 South Shore Drive
Chicago, Illinois 60637(US)
Inventor: Xiong, Yusheng
5532 South Kenwood Avenue
Chicago, Illinois 60637(US)
Inventor: Lee, Chih-Hung
822 East 57th Street
Chicago, Illinois 60637(US)

(74) Representative: Beresford, Keith Denis Lewis et al
BERESFORD & Co. 2-5 Warwick Court High Holborn
London WC1R 5DJ(GB)

# (54) Direct magnesiation of organic materials.

(57) A method for magnesiating a compound substituted with one or more activating groups beta (ortho) to a carbon atom having a hydrogen substituent to be deprotonated. The compound is either a saturated cyclic compound in which the carbon-carbon bonds are strained greater than the carbon-carbon bonds of cyclobutane, or an unsaturated compound in which the carbon atom to be deprotonated is located no more than one position away from a pi-bonded carbon atom. The method contacts the substituted compound with a reactant having the formula:

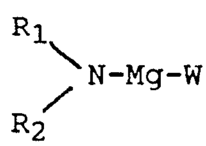

wherein W is:

Cl, Br, I or 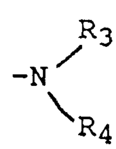

and $R_1$, $R_2$, $R_3$ and $R_4$ independently include hydrogen, alkyl, aryl, alkylaryl, and heteroaryl, or R and $R_2$ and/or $R_3$ and $R_4$ together form a cyclic residue. Compounds substituted with one or more magnesium-containing groups and one or more activating groups are also disclosed. The activated compounds are either saturated cyclic compounds having carbon-carbon bonds that are strained greater than the carbon-carbon bonds of cyclobutane, or unsaturated compounds. The one or more magnesium-containing groups have the structure:

EP 0 418 013 A2

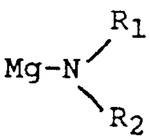

with $R_1$ and $R_2$ independently including hydrogen, alkyl, aryl, alkylaryl, and heteroaryl, or $R_1$ and $R_2$ together forming a cyclic residue. A method by which such compounds are reacted with electrophilic reagents replacing the magnesium containing groups with substituent groups is also disclosed.

# DIRECT MAGNESIATION OF ORGANIC MATERIALS

The present invention relates to a method for magnesiating compounds substituted with one or more activating groups having an unshared pair of electrons, which compounds are either unsaturated or strained saturated cyclic compounds, and to the resulting magnesiated compounds, stabilized by the one or more activating groups. The present invention is particularly useful in the magnesiation of such compounds in a position beta or ortho to the activating groups. The resulting beta (ortho) magnesiated products can then be reacted with electrophilic reagents to produce a number of useful beta (ortho) substituted products.

In the past, the substitution of groups beta (ortho) to substituents on unsaturated or strained saturated cyclic compounds has been obtained by first lithiating the compound beta (ortho) to the substituent group with a lithium base, such as a lithium amide. The lithium substituent could then be displaced by the desired functional group, or first replaced by another metal or metal salt, which could then be displaced by the desired functional group. Lithium amide bases lack versatility, however, because of their limited thermal stability in many solvents of use in metallation reactions. This limits the use of reactants of low solubility or low reactivity. A more stable reactant for metallating beta (ortho) to substituents on unsaturated or strained saturated cyclic compounds would be highly desirable.

It has now been discovered that substitution of groups beta (ortho) to activating substituents on unsaturated or strained saturated cyclic compounds can be obtained by directly magnesiating the compound with a magnesium base such as an amido magnesium halide or magnesium diamide. Both the magnesium bases and the resulting intermediate compounds are more stable at elevated temperatures than their lithium counterparts. For instance, magnesium amide bases are more stable than their lithium counterparts in refluxing tetrahydrofuran (THF), a commonly used solvent for such synthesis.

According to the present invention, unsaturated and strained saturated cyclic compounds substituted with activating groups having an unshared pair of electrons can be directly magnesiated beta (ortho) to the activating groups with amide magnesium halides ($R_2NMgX$) and magnesium diamides [$(R_2N)2Mg$]. While these reagents are known, they have been little used in organic syntheses, and their ability to directly magnesiate the above activated compounds has not been reported. With these reagents it is now possible to selectively beta (ortho) substitute the above activated compounds in high yield.

In accordance with the present invention, a process is also provided for magnesiating an organic compound substituted with one or more activating groups beta (ortho) to a carbon to be deprotonated, that carbon having at least one hydrogen on it. The activated compound is either a saturated cyclic compound with carbon-carbon bonds strained greater than the carbon-carbon bonds of cyclobutane, or an unsaturated compound in which the carbon to be deprotonated is located no more than one position away from a pi-bonded carbon atom. The process contacts the activated compound with a reactant having the formula:

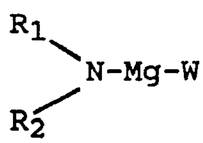

where W is selected from

Cl, Br, I and 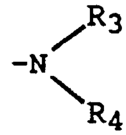

and $R_1$, $R_2$, $R_3$ and $R_4$ are independently selected from the group consisting of hydrogen, alkyl, aryl, alkylaryl, and heteroaryl, or $R_1$ and $R_2$, or $R_3$ and $R_4$, or both $R_1$ and $R_2$, and $R_3$ and $R_4$, together form a cyclic residue. Preferred processes will magnesiate the compound in at least one position beta (ortho) to at least one of the activating groups.

While not being bound by any particular theory, it is believed that the activating group having an unshared pair of electrons functions to promote deprotonation of carbons on the compound to be magnesiated, which carbons are beta (ortho) to the activating group, once the compound to be magnesiated is exposed to a strong base such as the magnesium amide bases. It is believed that the activating group

then further functions to stabilize the resulting magnesium substituted compound. The present invention further includes the additional discovery that magnesium amide bases are particularly suitable for the direct magnesiation of such compounds in that they provide the strong basicity necessary for deprotonation, as well as a readily substituted magnesiation group that once substituted is stabilized by the activating group. The combination of these two discoveries permits the direct magnesiation of the activated compounds by reaction with the magnesium amide bases in a single step reaction.

Other objects, features and advantages of the methods and compounds of the present invention will be more readily apparent from the detailed description of the preferred embodiment set forth below.

A magnesiation process according to one aspect of the present invention employs amide magnesium halides and magnesium diamides as direct magnesiation agents. These preferred magnesium amide bases can be expressed by the following formula:

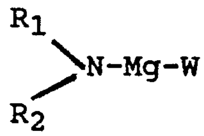

wherein W is selected from

Cl, Br, I and 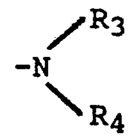

$R_1$, $R_2$, $R_3$ and $R_4$ are not critical and, for example, independently include, but are not limited to, hydrogen, alkyl, aryl, alkylaryl, and heteroaryl, or $R_1$ and $R_2$ and/or $R_3$ and $R_4$ together form a cyclic residue. Preferred Hauser bases include 2,2,6,6-tetramethylpiperidinomagnesium bromide and diisopropylamidomagnesium bromide. Preferred magnesium diamides include bis(2,2,6,6-tetramethylpiperidino)magnesium and bis (diisopropylamido)magnesium.

The magnesium amide bases are capable of deprotonating compounds having sufficiently acidic carbon-hydrogen bonds. Such compound must be substituted with one or more activating groups having an unshared pair of electrons beta (ortho) to the carbon atom from which the hydrogen atom is to be abstracted, and must be either saturated cyclic compounds in which the carbon-carbon bonds are strained greater than the carbon-carbon bonds of cyclobutane, or unsaturated compounds in which the carbon atom from which the hydrogen atom is to be extracted is itself a pi-bonded carbon atom or is adjacent to a pi-bonded carbon atom. The magnesium amide base then substitutes a magnesium-containing group onto the deprotonated carbon that forms.

The process of the present invention in most cases is selective for the beta (ortho) substitution of magnesium containing substituents on compounds substituted with activating groups. In particular, in processes in which the activated compound is a strained saturated cyclic compound or an unsaturated compound such as an aromatic compound, the process will be beta (ortho) selective. In processes in which the activated compound is an unsaturated alkene, rearrangement of the carbanion is possible and the process will not necessarily be beta (ortho) selective.

Any suitably activated saturated cyclic compound in which the carbon-carbon bonds are strained greater than the carbon-carbon bonds of cyclobutane is suitable for use in the process of the present invention. These saturated cyclic compounds may include other substituent groups in addition to the one or more activating groups. Preferred saturated cyclic compounds include suitably activated cyclopropanes and cubanes.

Any unsaturated compound in which the carbon to be deprotonated is located no more than one position away from a pi-bonded carbon atom is also suitable for use in the process of the present invention. The compound may include heteroatoms such as nitrogen or sulfur. A pi-bonded carbon atom is defined as a carbon atom bonded to other carbon atoms or to one or more heteroatoms in an aromatic structure or a carbon atom double bonded to another carbon atom or heteroatom. The unsaturated compounds may also include other substituent groups in addition to the activating groups.

One class of unsaturated compounds suitable for use in the present invention are aromatic compounds. By definition, the atoms of an aromatic structure are pi-bonded to each other. Therefore, any carbon atom to be deprotonated on an aromatic structure will be a pi-bonded carbon atom. The activated aromatic ring is

susceptible to deprotonation at positions ortho to the activating group by the magnesium amide base and the hydrogen is replaced by a magnesium substituent at this position. The process of the present invention is therefore particularly useful for the high-yield ortho-selective substitution of suitably activated aromatic compounds. Aromatic compounds such as benzene and substituted benzenes are suitable for use in the present invention as well as heterocyclic aromatic compounds and polycyclic aromatic compounds.

Multiply-activated aromatic rings are also suitable for use in the present invention and may be magnesiated ortho to both activating groups resulting in, for example, magnesiation group substitution of benzene rings at the two- and five- positions for benzene rings activated in the one- and four- positions, and magnesiation group substitution of benzene rings at the three- and six- positions for benzene rings activated in the one- and two- positions. The lithiation reactions of the prior art were unable to achieve double ortho substitution on a double-activated aromatic ring without resorting to halogen-metal exchange reactions or reverse transmetalation processes. The process of the present invention now makes possible direct multiple ortho substitution of a multiply-activated aromatic ring.

Another class of unsaturated compounds suitable for use in the present invention are alkenes in which the carbon atom to be deprotonated is located no more than one position away from a double-bonded carbon, which by definition is a pi-bonded carbon, and beta to a carbon atom having an activating group. Both straight chain and cyclic alkenes are suitable for use in the present invention, and may include one or more heteroatoms. Preferred cycloalkenes includes cyclohex-3-enes substituted in the one-position with an activating group. As stated above, unlike strained saturated cyclic compounds and aromatic rings, reacting alkenes and cycloalkenes with magnesium amide bases will not always result in substitution directly beta to the activating group. Rearrangement of the intermediate carbanion can occur and the magnesiated end product that is formed will depend upon the rate at which substitution at each position on the rearranged alkene occurs and will vary with the starting material.

Any substituent group capable of assisting the deprotonation of a carbon atom beta (ortho) to it is suitable for use as an activating group for the compounds to be magnesiated by the process of the present invention. Such substituent groups include by way of example, but are not limited to, those described by Beak et al., Acc. Chem. Res. , 15 , 306 (1982). Preferred activating groups include amino-carbonyls and thio-analogs thereof, oxazolines, imides, amines, carbamates, nitriles, esters, amino alkyls, hydroxyalkyls, halogenated methyls, sulfonamides, alkyl-amines, ethers, ketals, acetals, nitros and sulfonates.

When more than one activating group is substituted on the compound to be magnesiated, such activating groups may be the same or different. When more than one activating group is substituted on an aromatic ring, the relative ability of the functional groups to activate for ortho magnesiation will vary. For example, when p-carbomethoxy-N,N-diethylbenzamide is reacted in room temperature THF with an equimolar amount of bis(2,2,6,6-tetramethylpiperidino) magnesium ($(TMP)_2Mg$) substitution ortho to the carbomethoxy group over the amide group predominates by a ratio of about 3:1. Once an excess of $(TMP)_2Mg$ is used, however, the 2,5- substituted reaction product is obtained along with the single substituted products.

The process of the invention is carried out by contacting the compound to be magnesiated, substituted with one or more activating groups, with a magnesium amide base. The two materials are preferably contacted at room temperature for the minimum time necessary to provide the desired yield, preferably less than four hours. The reaction temperature chosen should be one in which the magnesium amide base is stable. These bases have proven stable at the temperature of refluxing THF, about 70° C, and higher. Temperatures between about 0 and about 50° C are preferred. Higher and lower reaction temperatures are also suitable. Many common solvents are useful, provided that the solvent should not have acidic protons that are more acidic than that of the amine from which the magnesium amide base is derived. For example, THF, diethyl ether and toluene are all suitable solvents, while water and methanol and other alcohols would not be suitable.

The reaction product will be a compound substituted with one or more magnesium-containing groups and one or more activating groups having an unshared pair of electrons. The compound is either a saturated cyclic compound having carbon-carbon bonds strained greater than the carbon bonds of cyclobutane or an unsaturated compound, with the magnesium-containing groups having the structure:

Mg-W

When an amide magnesium halide is used in the process of the invention as the magnesium amide base, W for the reaction product is Cl, Br or I. When a magnesium diamide is used in the process of the invention as a magnesium amide base, W for the reaction product is

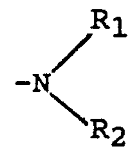

and the reaction products are novel compounds. The substituent groups of $R_1$ and $R_2$ are not critical and, for example, independently include, but are not limited to, hydrogen, alkyl, aryl, alkylaryl, and heteroaryl, or $R_1$ and $R_2$ together form a cyclic residue. Except for instances of a rearrangement of alkene compounds, the magnesium containing groups will be located beta (ortho) to the one or more activating groups. When more than one activating group is present, the groups may be the same or different. Likewise, when the product is substituted with more than one magnesium-containing group, by using more than one magnesium amide base, the groups substituted may be the same or different.

The saturated strained cyclic reaction products hereof include cyclopropane and cubane derivatives. The unsaturated reaction products include aromatic compounds, including polycyclic and heterocyclic aromatic compounds, and alkenes including cycloalkenes. As indicated above, with alkenes and cycloalkenes, the position of the one or more magnesium-containing groups relative to the one or more activating groups and the pi-bonded carbons on the alkene may vary because of rearrangement of the intermediate carbanion. Otherwise, with aromatic and saturated strained cyclic compounds, the one or more magnesium containing groups will be substituted beta (ortho) to an activating group.

Particularly useful reaction products include single activated, single ortho magnesiated aromatic ring compounds having the formula:

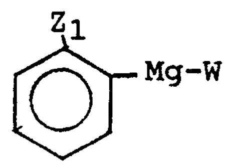

and double double ortho magnesiated aromatic rings having the formulae:

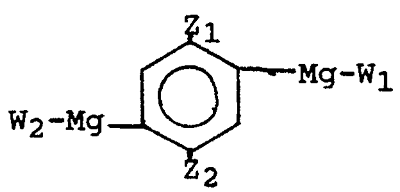

and

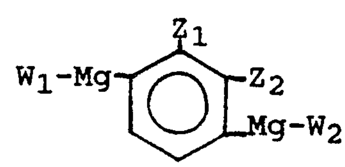

Again, when an amide magnesium halide base is used in the process of the invention as the magnesium amide base, $W_1$ and $W_2$ for the reaction product are Cl, Br or I. When a magnesium diamine is used as the magnesium amide base, $W_1$ and $W_2$ for the reaction product are

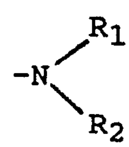

and the reaction products are novel compounds. $R_1$ and $R_2$ independently include hydrogen, alkyl, aryl, alkylaryl, and heteroaryl, or $R_1$ and $R_2$ together form a cyclic or polycyclic residue. $Z_1$ and $Z_2$ may be any activating group and may be the same or different, and include by example, but are not limited to, aminocarbonyls and thio-analogs thereof, oxazolines, imides, amines, carbamates, nitriles, esters, aminoalkyls, hydroxyalkyls, halogenated methyls, sulfonamides, alkyl-amines, ethers, ketals, acetals, nitros and sulfonates.

The magnesium substituted reaction products of the invention may be further reacted with electrophilic

reagents to substitute the electrophilic group of the reagent for the magnesium containing group of the reaction product. Examples of electrophilic reagents suitable for use in this invention are as follows, with the respective electrophilic group introduced listed parenthetically: $D_2O$ or deuterated alcohols having the formula R-O-D (deuterium), metal salts (metal, metal salt), sulfonyl halides (sulfone), aldehydes and ketones (alcohols), carbon dioxide (carboxylic acid magnesium salt), acid chlorides, acid anhydrides and acid esters (ketones and/or tertiary alcohols), formamides (carboxaldehydes), silyl halides (silanes), borates/hydrogen peroxide (hydroxyl), cyanates (carbamates), alkyl, aryl and alkylaryl agents (alkyl, aryl or alkylaryl), bromine (bromide), chlorine (chloride), iodine (iodide), nitrites or nitrosyl halides (nitroso), disulfides (sulfides), azides/borohydrides (amines), N-fluoro-N-sulfonamides (fluoro), methoxamines/methyllithiums (amines), boron trichloride (boron halide/borates), o-(diphenylphosphinyl) hydroxylamines (primary, secondary or tertiary amines), phosphorous and phosphoryl halides (phosphorous derivatives), dinitrogen tetroxide (nitro), 1-(benzotriazol-1-yl)alkylamine (aminoalkyl) and 1-(trimethylsilyl) vinylphosphonates (beta-ketophosphonate).

When the electrophilic reagent is carbon dioxide, carboxylic acid magnesium salt groups are substituted for the magnesium-containing groups, which can then be acidified to form carboxylic acid groups. The carboxylic acid groups can be further reacted by the known methods to form carboxylic acid esters and other functional derivatives. Typical methods include reacting the acid with an alcohol, converting the acid into an acid chloride that is then reacted with an alcohol to form the acid ester, or reacting the acid with a diazoalkyl reagent.

Except for alkenes that have undergone carbanion rearrangement, the process of this invention ultimately results in high yields of compounds having substituents beta (ortho) to activating groups having an unshared pair of electrons. This process is particularly useful in the ortho substitution of aromatic rings. Thus, it can be appreciated that the direct magnesiation process of the present invention provides a versatile method for syntheses by direct magnesiation of compounds based upon saturated strained cyclic or unsaturated compounds substituted with one or more activating groups having an unshared pair of electrons.

The following examples are illustrative of the application of the process of the present invention and are not to be construed as limiting the scope thereof.

## EXAMPLES

### Example 1

### o-Magnesiation of N.N-Diethylbenzamide

6 ml of a 0.5 molar solution of bis (2,2,6,6-tetramethylpiperidino) magnesium ($(TMP)_2Mg$) in tetrahydrofuran (THF) was added dropwise to two milliliters of a 0.57 mmol solution of N,N-diethylbenzamide in THF (50 mg/ml). The reaction mixture was stirred at room temperature for two hours. The solution was cooled in a dry ice/acetone bath and dry carbon dioxide was bubbled through. The cooling bath was removed after forty minutes. The pale yellow reaction mixture was warmed to room temperature. Addition of carbon dioxide was discontinued and the solution was acidified to a pH of about 1-2 with 0.1 M hydrochloric acid. The solution was extracted with ethyl acetate (3 x 15 ml) and the aqueous layer was discarded. The organic layer was carefully extracted with 10% $Na_2CO_3$ and then discarded. The base extract was acidified with concentrated hydrochloric acid to a pH of about 1-2, and then extracted again with ethyl acetate (3 x 15 ml). The organic extract was dried over $Na_2SO_4$. The solvent was removed in vacuum leaving 129 mg of crude acid. Crystallization from ethyl acetate gave a white solid with a melting point of 150-152 °C identified by $^1H$-NMR and $^{13}C$-NMR as pure o-carboxy-N,N-diethylbenzamide. The yield was 110 mg or 87%. Esterification with diazomethane in ether gave a compound identified by the above NMR techniques as o-carbomethoxy-N,N-diethylbenzamide.

### Example 2

o-Magnesiation of Methylbenzoate

6 ml. of a 0.5 M solution of $(TMP)_2Mg$ in THF was added dropwise to two ml of a 0.74 mmol solution of methylbenzoate in THF (50 mg/ml) at room temperature. The reaction mixture was stirred at room temperature for 45 minutes. Dry carbon dioxide was bubbled through the solution at dry ice/acetone bath temperature for forty minutes. The cooling bath was removed, and the pale yellow reaction mixture was warmed to room temperature. Addition of carbon dioxide was discontinued, and the solution was acidified to a pH of about 1-2 with 0.1 M hydrochloric acid. The reaction product was removed from the reaction mixture by extraction as in Example 1. Once the solvent was removed in vacuum, 119 mg of crude acid remained. Crystallization from pure hexanes gave 110 mg of a white solid having a melting point of 82-84 °C. Application of the NMR techniques of Example 1 and mass spectroscopy identified the material as pure o-carboxy-methylbenzoate. The yield was 87%. Esterification with diazomethane gave a liquid having a boiling point of 282-284 °C identified by the NMR techniques of Example 1 and mass spectroscopy as dimethyl o-phthalate.

Example 3

Di-o-Magnesiation of N,N-Diethylterephthalamide

5 ml. of a 0.5 M solution of $(TMP)_2Mg$ in THF was added dropwise to two milliliters of a 0.36 mmol solution of N,N-diethylterephthalamide in THF (50 mg/ml) at room temperature. The reaction mixture was refluxed for two hours. Dry carbon dioxide was then bubbled through the solution at a dry ice/acetone bath temperature for forty minutes. The cooling bath was removed and the pale yellow reaction mixture was warmed to room temperature. Addition of the carbon dioxide was discontinued and the solution was acidified to a pH of about 1-2 with 0.1 M hydrochloric acid. The reaction product was removed from the solution by extraction as in Example 1 and once the solvent was removed in vacuum, 120 mg of crude acid remained. Crystallization from ethyl acetate gave 116 mg of a white solid identified by the NMR techniques of Example 1 as pure 3, 6-dicarboxy-N,N-diethylterephthalamide. The yield was 88%. Esterification with diazomethane in ether gave a compound having a melting point of 148-150 °C identified by the NMR techniques of Example 1 as 3, 6-dicarbomethoxy-N,N-diethylterephthalamide.

Example 4

Preparation of Cis-2-Carboxy-1-Methyl-(N,N-diisopropyl-carboxamido) Cyclopropane

6 ml. of a 0.5 M solution of $(TMP)_2Mg$ in THF was added dropwise to two milliliters of a 0.55 mmol solution of 1-methyl-(N,N-diisopropylcarboxamido) cyclopropane in THF (50 mg/ml) at room temperature. The reaction mixture was refluxed for one hour. Dry carbon dioxide was bubbled through the solution at dry ice/acetone bath temperature for forty minutes. The bath was removed and the pale yellow reaction mixture was warmed to room temperature. Addition of carbon dioxide was discontinued. The solution was acidified to a pH of about 1-2 with 0.1 M hydrochloric acid. The reaction product was removed from the solution as in Example 1 by extraction. After the solvent was removed in vacuum 119 mg of crude acid remained. Crystallization from hexanes gave 119 mg of a white solid having a melting point of 142-144 °C identified by the NMR techniques of Example 1 as pure cis-2-carboxy-1-methyl-(N,N-diisopropylcarboxamido) cyclopropane. The yield was 88%.

Example 5

Preparation of 2, 7-Dicarbomethoxy-1, 4-bis (N,N-diisopropyl-carboxamido) cubane

5 ml. of a 0.5 M solution of $(TMP)_2Mg$ in THF was added dropwise to two milliliters of a 0.28 mmol solution of 1,4-bis-(N,N-diisopropylcarboxamido) cubane in THF (50 mg/ml) at room temperature. The reaction mixture was refluxed for three hours. Dry carbon dioxide was then bubbled through the solution at dry ice/acetone bath temperature for forty minutes. The bath was removed and the pale yellow reaction mixture was warmed to room temperature. Addition of carbon dioxide was discontinued. The solution was acidified to a pH of about 1-2 with 0.1 M hydrochloric acid. The reaction product was removed from the solution by extraction as in Example 1 and the solvent was removed in vacuum with 110 mg of crude acid remaining. Crystallization from ethyl acetate gave 109 mg of a white solid identified by the NMR techniques of Example 1 as pure 2,7-dicarboxy-1,4-bis(N,N-diisopropylcarboxamido)cubane. The yield was 85%. Esterification with diazomethane in ether gave a solid having a melting point of 206-208 °C identified by the NMR techniques of Example 1 as 2,7-dicarbomethoxy-1,4-bis(N,N-diisopropylcarboxamido)cubane.

## Example 6

Preparation of 2,4-bis(trifluoromethyl)benzoic acid

6 ml of a 0.5 M solution of bis (diisopropylamido) magnesium in dry THF was added dropwise to two milliliters of a 0.57 mmol solution in THF at room temperature of 1,3-bis(trifluoromethyl) benzene. The mixture was stirred at room temperature for 45 minutes. The solution was then cooled in a dry ice/acetone bath to approximately -70 °C, and dry carbon dioxide was bubbled through. The cooling bath was removed after 40 minutes. The brown reaction mixture was warmed to room temperature. Addition of carbon dioxide was discontinued. The solution was acidified to a pH of about 1-2 with 0.1 M hydrochloric acid. The solution was extracted with chloroform (3 x 15 ml) and the aqueous layer was discarded. The organic layer was extracted carefully with aqueous 10% NaOH, then discarded. The base extract was acidified with concentrated hydrochloric acid to a pH of about 1-2, and then extracted again with chloroform (3 x 15 ml). The organic extract was dried over $Na_2SO_4$. The solvent was removed in vacuum, leaving 123 mg of solid crude acid, which contained 2,4-bis(trifluoromethyl)benzoic acid and 3,4-bis(trifluoromethyl)benzoic acid in a 93:7 ratio. Crystallization from hexanes gave 98 mg of colorless prisms having a melting point of 108-110 °C identified by the NMR techniques of Example 1 as pure 2,4-bis(trifluoromethyl)benzoic acid. The yield was 81%.

As will be readily appreciated, numerous variations and combinations of the features set forth above can be utilized without departing from the present invention as set forth in the claims. Such variations are not regarded as a departure from the spirit and scope of the invention, and all such modifications are intended to be included within the scope of the following claims.

## Claims

1. A method of magnesiating an organic compound substituted with one or more activating groups beta or ortho to a carbon atom having a hydrogen substituent to be deprotonated, said substituted compound being selected from the group consisting of saturated cyclic compounds including carbon-carbon bonds strained greater than the carbon-carbon bonds of cyclobutane, and unsaturated compounds in which said carbon atom to be deprotonated is located no more than one position away from a pi-bonded carbon atom, said method characterized by contacting said substituted compound with a reactant having the formula:

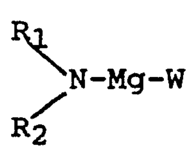

wherein W is selected from the group consisting of:

Cl, Br, I and 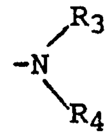

and $R_1$, $R_2$, $R_3$ and $R_4$ are independently selected from the group consisting of hydrogen, alkyl, aryl, alkylaryl, and heteroaryl, or $R_1$ and $R_2$, or $R_3$ and $R_4$, or both $R_1$ and $R_2$, and $R_3$ and $R_4$ together form a cyclic residue.

2. The method of claim 1, characterized in that said substituted compound and said reactant are contacted at a temperature between about 0° C and about 50° C.

3. The method of claim 1 or 2, characterized in that said temperature is about room temperature.

4. The method of claim 1, 2, or 3, characterized in that said one or more activating groups are selected from the group consisting of amino-carbonyls and thio-analogs thereof, oxazolines, imides, amines, carbamates, nitriles, esters, aminoalkyls, hydroxyalkyls, halogenated methyls, sulfonamides, alkyl-amines, ethers, ketals, acetals, nitros and sulfonates.

5. The method of claim 1, 2, or 3, characterized in that said substituted compound is substituted with two activating groups that are the same or different.

6. The method of claim 1, 2, or 3, characterized in that said substituted compound includes one or more heteroatoms.

7. The method of claim 1, 2, or 3, characterized in that said substituted compound comprises a saturated cyclic compound selected from the group consisting of cyclopropane derivatives and cubane derivatives.

8. The method of any one of the previous claims, characterized in that said substituted compound comprises an unsaturated compound selected from the group consisting of aromatic compounds and alkenes in which the carbon atom to be deprotonated is located no more than one position away from a double-bonded carbon atom.

9. The method of claim 8, characterized in that said alkene comprises a cyclic alkene.

10. The method of claim 9, characterized in that said cyclic alkene comprises cyclohex-3-ene.

11. The method of claim 7, characterized in that said aromatic compound comprises N,N-diethylterephthalamide.

12. The method of claim 1, 2, or 3, characterized in that said reactant comprises a magnesium amide base selected from the group consisting of bis(2,2,6,6-tetramethylpiperidino) magnesium, bis(diisopropylamido) magnesium, 2,2,6,6-tetramethylpiper-idinomagnesium bromide and diisopropylamidomagnesium bromide.

13. The method of any one of the previous claims further characterized by the step of reacting said magnesiated compound with an electrophilic reagent.

14. The method of claim 13, characterized in that said electrophilic reagent is selected from the group consisting of $D_2O$, deuterated alcohols, metal salts, sulfonyl halides, aldehydes, ketones, carbon dioxide, acid chlorides, acid anhydrides, acid esters, formamides, silyl halides, borates with hydrogen peroxide, cyanates, alkyl, aryl and arylalkyl agents, chlorine, bromine, iodine, nitrites, nitrosyl halides, disulfides, azides with boron hydrides, N-fluoro-N-sulfonamides, methoxyamines with methyllithium, boron trichloride, dinitrogen tetroxide, O-(diphenylphosphinyl) hydroxylamines, phosphorus, phosphoryl halides, 1-(benzotriazol-1-yl)alkylamines, and 1-(trimethylsilyl) vinylphosphonates.

15. The method of claim 14, characterized in that said electrophilic reagent comprises carbon dioxide whereby a carboxylic acid magnesium salt results therefrom, and wherein said method further comprises the step of forming a carboxylic acid derivative from said carboxylic acid magnesium salt.

16. A compound substituted with one or more magnesium-containing groups and one or more activating groups, which compound is selected from the group consisting of saturated cyclic compounds including carbon-carbon bonds strained greater than the carbon-carbon bonds of cyclobutane and unsaturated compounds, said compound characterized by said one or more magnesium containing groups have the structure:

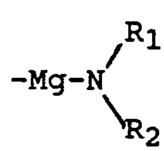

wherein $R_1$ and $R_2$ are independently selected from the group consisting of hydrogen, alkyl, aryl, alkylaryl, and heteroaryl, or $R_1$ and $R_2$ together form a cyclic residue.

17. The compound of claim 16, characterized in that said one or more activating groups are independently selected from the group consisting of amino-carbonyls and thio-analogs thereof, oxazolines, imides, amines, carbamates, nitriles, esters, aminoalkyls, hydroxyalkyls, halogenated methyls, sulfonamides, alkyl-amines, ethers, ketals, acetals, nitros and sulfonates.

18. The compound of claim 16 or 17, characterized in that said compound is substituted with two magnesium-containing groups that are the same or different.

19. The compound of claim 16, 17, or 18, characterized in that said compound includes one or more heteroatoms.

20. The compound of claim 16, 17, or 18, characterized in that said saturated cyclic compound has carbon-carbon bonds strained greater than the carbon-carbon bonds of cyclobutane is substituted with at least one magnesium containing group in a position beta to said at least one activating group

21. The compound of claim 16, 17, or 18, characterized in that said saturated cyclic compound has carbon-carbon bonds strained greater than the carbon-carbon bonds of cyclobutane selected from the group consisting of cyclopropane derivatives and cubane derivatives.

22. The compound of claim 16, characterized by said compound comprising an unsaturated compound selected from the group consisting of aromatic compounds and alkenes.

23. The compound of claim 22, characterized in that said alkene comprises a cyclic alkene.

24. The compound of claim 23, characterized in that said cyclic alkene comprises a cyclohexene.

25. The compound of claim 22, characterized in that said aromatic compound is substituted with at least one magnesium-containing group in a position ortho to said at least one activating group.

26. The compound of claim 21, characterized in that said compound has the formula:

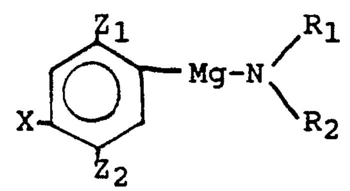

wherein $Z_1$ comprises an activating group, $Z_2$ comprises hydrogen or an activating group, X comprises hydrogen or:

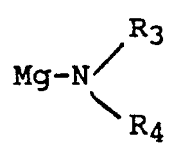

and $R_1$, $R_2$, $R_3$ and $R_4$ are independently selected from the group consisting of hydrogen, alkyl, aryl, alkylaryl, and heteroaryl, or $R_1$ and $R_2$, or $R_3$ and $R_4$, or both $R_1$ and $R_2$ and $R_3$ and $R_4$ together form cyclic residues.

27. The compound of claim 21, characterized in that said compound has the formula:

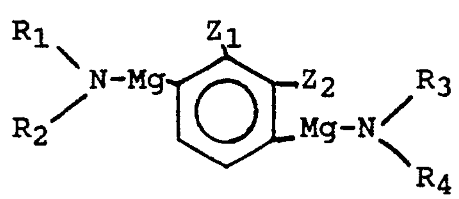

wherein $Z_1$ and $Z_2$ comprise activating groups, and $R_1$, $R_2$, $R_3$ and $R_4$ are independently selected from the group consisting of hydrogen, alkyl, aryl, alkylaryl and heteroaryl, or $R_1$ and $R_2$, or $R_3$ and $R_4$, or both $R_1$ and $R_2$ and $R_3$ and $R_4$ together form cyclic residues.

28. The compound of claim 26 or 27, characterized in that $Z_1$ and $Z_2$ comprise activating groups

independently selected from the group consisting of amino-carbonyls and thio-analogs thereof, oxazolines, imides, amines, carbamates, nitriles, esters, aminoalkyls, hydroxyalkyls, halogenated methyls, sulfonamides, alkyl-amines, ethers, ketals, acetals, nitros and sulfonates.

29. The use of a magnesium amide base for direct magnesiation of a compound having an acidic carbon-hydrogen bond.

30. Use of a magnesium amide base according to claim 29, in which said compound is substituted with one or more activating groups having an unshared pair of electrons beta (ortho) to the acid carbon-hydrogen bond.

31. Use of a magnesium amide base according to claim 29 or 30, in which said compound is a saturated cyclic compound in which a carbon-carbon bond is strained greater than those of cyclobutane or is a compound having a pi-bond that affects the carbon of the acidic carbon-hydrogen bond.